# EUROPEAN PATENT APPLICATION

(11) **EP 0 627 625 A1**
(43) Date of publication of application: **07.12.1994**
(21) Application number: 93109005.4
(22) Date of filing: 04.06.1993
(51) Int. Cl.: G01N 33/558, G01N 33/569, G01N 33/543

(54) **Immunoassay for differential diagnosis**

(71) Applicant: ABBOTT LABORATORIES, Abbott Park, Illinois 60064-3500 (US)
(72) Inventor: Simpson, Barry, Dr., 6204 Taunusstein 4 (DE); Michel, Gerd, Dr., 6200 Wiesbaden (DE); Gultekin, Halil, Dr., 6229 Schlangenbad-5 (DE); Sutherland, Ranald, Dr., 6200 Wiesbaden (DE)
(74) Representative: Modiano, Guido, Dr.-Ing.

(57) **Abstract**

An improved Western blot method wherein the captured antibody of a test sample is detected by a probe specific for the captured antibody. The capture reagent can be a lysate, a recombinant protein or a synthetic peptide specific for the antibody to be detected. A test kit for performing the method also is provided.

## Description

### Background of the Invention

This invention relates generally to a method for determining the presence of a specific binding pair member, preferably an antibody, in a test sample, and more particularly, relates to the detection of a member of a specific binding pair in a test sample by an improved Western Blot assay.

The human immune system responds to infections by generating antibodies to various antigens. The number of antibodies and the amount of each different antibody produced depends on the infectious agent and the particular antigen(s) which initiate the antibody (immune) response. The detection of an immune response to an infectious agent is an important method in *in vitro* diagnostics. This method is especially useful when detecting antibodies against these infectious agents in donated blood units, and when testing body fluids for the presence of these agents in order to diagnose acute and chronic infections. Such tests typically are initially performed by contacting a test sample such as a body fluid suspected of containing antibody to the agent of interest with a known antigen(s) obtained from the agent of interest. The antigen(s) usually is immobilized on a solid phase material to allow ease of separation from the test sample and for further treatment. Following incubation of the solid phase with the test sample, unbound materials are removed, and any bound antibody usually is detected by a second incubation with a specific anti-human immunoglobulin labelled with a detectable measurable label (a so-called conjugate). Following incubation, unbound materials are removed and the bound labelled complex is detected as a measure of the detectable signal. The antigens used in these initial tests (or so-called screening tests) may employ purified lysates or recombinant proteins.

These screening tests, however, have known non-specific reactivities. See, for example, J. E. Menetove et al., Lancet:1213 (November 21, 1987); D. Barnes, Science 238:884-885 (1987); A. Puckett et al., Lancet:714 (March 26, 1988). Some of the non-specificity is attributable to immunoreactivities with normal human antigens that are present in these lysates of the infectious agent. The normal human antigens (cellular protein impurities) originate from the tissue culture cells or other culture media that are used to propagate the infectious agent, and they are the major constituents of partially purified lysates. For example, the major surface antigen of Human Immunodeficiency Virus (HIV), gp120, and the HIV transmembrane antigen gp41 are readily lost during viral purification. H. Gelderblood et al., Virology 156:171-176 (1987). These HIV envelope antigens thus often are underrepresented in viral lysates. Since non-specific reactions are known to occur with screening tests, for example the HIV screening tests, a confirmatory test usually is required to corroborate reactive results obtained with these screening tests.

The protocol for performing such confirmatory tests typically involves repeating the screening test to ensure a reactive test sample, and then performing a Western blot (WB) test on these repeat reactive test samples to confirm the reactive screening result. In order to be considered reactive by a Western blot test, reactivity to more than one antigen usually is required. Thus, reactivity to a single antigen is not sufficient to confirm a "true" antibody positive sample. Only those test samples which also are reactive by the WB test are considered as confirmed positive for the infectious agent tested.

The typical Western blot test is described by Gordon et al., U.S. Patent No. 4,452,901. This test initially involves separating the antigens of a viral lysate according to size by SDS-PAGE under reducing conditions. The invisible bands of antigens in the gel then are electrophoretically transferred (transblotted) intact onto a sheet of nitrocellulose. This sheet then is cut into strips perpendicular to the bands. The strips are allowed to react with individual test samples, and the reactive antibodies are detected by employing an anti-globulin conjugate and suitable enzyme label. The resultant color reaction yields a pattern of stained bands revealing those antigens for which the test sample is seropositive. The WB test therefore has an advantage over screening test because the antigens in the viral lysate which are reactive with the individual antibodies can be visually separated. Additionally, non-viral reactivities in the inter-band areas can be identified and disregarded.

Despite the recited advantages of the WB test over traditional screening assays, the WB test has its drawbacks. See, for example, G. Biberfeld et al., Lancet ii:289-290 (1986); C. L. Van de Poel et al., Lancet ii:752-754 (1986); and A. M. Courouce, Lancet ii:921-922 (1986). Traditional immunoblot methods such as the WB test lack reproducibility because viral lysates may vary in antigenic concentration. Further, the WB test reportedly lacks sensitivity, particularly in regard to the HIV envelope (ENV) antigens. See, for example, A Saah et al., J. Clin. Micro. 259:1605-1610 (1987); P.P. Mortimer, Lancet 337:286-87 (1991); and F. Simon et al., Lancet 340:1541-1542 (1992). The WB test also yields a number of non-specific reactions and falsely positive elevated results if the test sample's antibodies react non-specifically with either the immobilized proteins or the solid phase matrix. Further, extra bands of reactivities that partly are due to partially cleaved or uncleaved viral precursor proteins and minor bands of reactivity may be present due to the presence of regulatory proteins. See, for example, S. Zolla-Pazner et al., New Engl. J. Med. 320:18-19 (1989). Further, elevated background signals can be found with a conjugate such as an anti-human globulin, which can bind non-specifically with the matrix material itself. This non-specific signal effectively limits the sensitivity of most anti-globulin testing methods as detection is limited by the concentration of conjugate that can be used; the useful concentration of conjugate is limited by the background signal. Also, the use of anti-globulin conjugates and, in particular, monospecific antibodies, can result in less than optimal signals where the conjugate does not detect all classes of globulin with equal affinity. Further, the WB test cannot differentiate between different specificities of the antibody, limiting the technique to a general anti-globulin detection method. The use of recombinant proteins in place of viral lysates can prevent the problems associated with using such lysates; however, the presence of new impurities and degraded antigenic fragments may cause different problems to occur. See, for example, U. S. Patent No. 5,120,662, which enjoys common ownership and is incorporated herein by reference.

Attempts to improve the standard WB technology have been attempted. For example, GB 2193315A teaches that methods of detecting antibodies to retroviruses are subject to false positive and false negative results, and describes an improved WB blot method where the signal producing system is a two-component system which can improve performance. In addition, EPA 296398 A1 describes an Improved WB method where a metal colloid labelled protein A is used to detect the immobilized sample antibodies. EP 397129 further discloses a WB immunoassay involving the simultaneous transfer of multiple antigens and/or antibodies onto a single solid support.

The use of several antigens in a single assay has been described. Lin et al., J. Virol. 59:522-524 (1986) describes a dual antibody probing technique that permitted identification of Epstein-Barr virus and different herpes virus antigens in the same Western Blot test. It also is possible to use multiple detection methods on a single Western blot test. Lee et al., J. Immuno. Methods 106:27-30 (1988) describes a technique which uses sequentially applied sets of probing antibodies, enzyme-conjugated developing antibodies and enzyme substrates to detect two or more types of interferon on a single Western blot test. The same result can be achieved by simultaneously applying more than one type of probing antibody using a mixture of different enzyme-conjugated developing antibodies followed by successive applications of different substrates.

Gordon et al., European Patent Application Publication No. 0 063 810, published March 11, 1982, describe immunoassay devices and kits comprising antigens or antibodies or both bound to a solid support. The use of the described solid supports makes possible a number of simultaneous antibody-antigen reactions in one operation. Gordon et al. describe applying single or successive doses of solutions of antigens or antibodies to the surface of the solid support using a pipet or a syringe. In a preferred embodiment, the antigen is applied as a microdot formed by adding small volumes of an antigenic solution.

Lefkovits, WO 87/03965, published July 2, 1987, describes a test strip for several simultaneous assays. The test strip is made from nitrocellulose impregnated with an antibody that is cut into strips and mounted on an inert backing. The soaking of a support sheet in a solution of antigenic peptide and then cutting the sheet into strips also is described.

U.S. Patent No. 5,061,619 describes the use of an antigen:labelled antibody complex to detect a single specifically immobilized antibody. However, the antigen has to be sufficiently large (i.e., a macromolecule) so that the formation of the labelled antibody:antigen complex does not prevent the binding of the complex by the antibody of interest This described method thus has a very limited use only to macromolecular antigens which have repeating epitopes. Further, it is not particularly well-suited to most antibody-specific assays, especially those that use small antigens, are based on oligopeptides and/or those that use non-repeating epitopes.

Thus, there is a need to provide a means for specifically and sensitively detecting different antibody populations, particularly in the area of viral infections. While the current art has attempted to fill the void, these various attempts have not been completely successful.

### Summary Of The Invention

The present invention provides a method for detecting one or more specific antibodies suspected of being present in a test sample, comprising:(a) contacting the test sample with a matrix containing more than one antigen, wherein said antigens are spatially physically separated from each other on said matrix, for a time and under conditions sufficient to form antigen/antibody complexes; (b) contacting said antigen/antibody complexes of step (a) with an indicator reagent wherein said indicator reagent comprises a probe specific for the antibody conjugated to a signal generating compound capable of generating a detectable measurable signal; and (c) detecting a measurable signal as an indication of the presence of the antibody in the test sample. The antigen and probe each may comprise a lysate, a recombinant protein or a synthetic peptide. In a preferred embodiment to detect HIV specific antibodies, the antigens may be selected from the group consisting of HIV-1 p24, HIV-1 gp41, HIV-1 p41, HIV-2 gp36 and HIV-2 p36. The invention is useful for detecting antibodies not only against HIV but also for detecting antibodies against any retrovirus or a hepatotrophic virus. In the latter case the present invention is particularly useful for detecting and identifying antibodies against HBV and HCV. It is contemplated in another embodiment that each indicator reagent capable of detecting each antigen is labelled with a different signal generating compound and used in the method, and that these signal generating compounds can be selected from the group consisting of a luminescent compound, a chemiluminescent compound, an enzyme, a colorimetric or a chromogenic compound. The signal generating compound further can comprise a hapten/anti-hapten binding pair, such as biotin/anti-biotin, biotin/steptavidin, fluorescein/anti-fluorescein binding pairs.

A test kit for detecting one or more antibodies in a test sample also is provided. The test kit comprises (a) a capture antigen specific for the antibody to be detected; and (b) an indicator reagent comprising a probe specific for the antibody to be detected conjugated to a signal generating compound capable of generating a detectable measurable signal. The antigen may be a lysate, a recombinant protein or a synthetic peptide. In a preferred mode, the lysate may be an HIV lysate containing antigens selected from the group consisting of HIV-1 p24, HIV-1 gp41, HIV-1 p41, HIV-2 p36 and HIV-2 p36. Further, in the case of HCV antibody detection, antigens representing the structural and non-structural genomic regions are preferred. For example, HCV antigens to be core, 33c, c-100, E1/E2 and NS5 region are preferred. The signal generating compound of the indicator reagent can be a luminescent compound, a chemiluminescent compound, an enzyme or a chromogenic compound.The signal generating compound further can comprise a hapten/anti-hapten binding pair, such as biotin/anti-biotin, biotin/steptavidin, fluorescein/anti-fluorescein binding pairs.

### Brief Description of the Drawings

FIGURES 1 and 2 are flow diagrams comparing the method of the present invention ("invention") to that of the prior art ("standard method").

FIGURES 3, 4, 5, and 6 are photographs of blots obtained by the method of the present invention.

### Detailed Description of the Invention

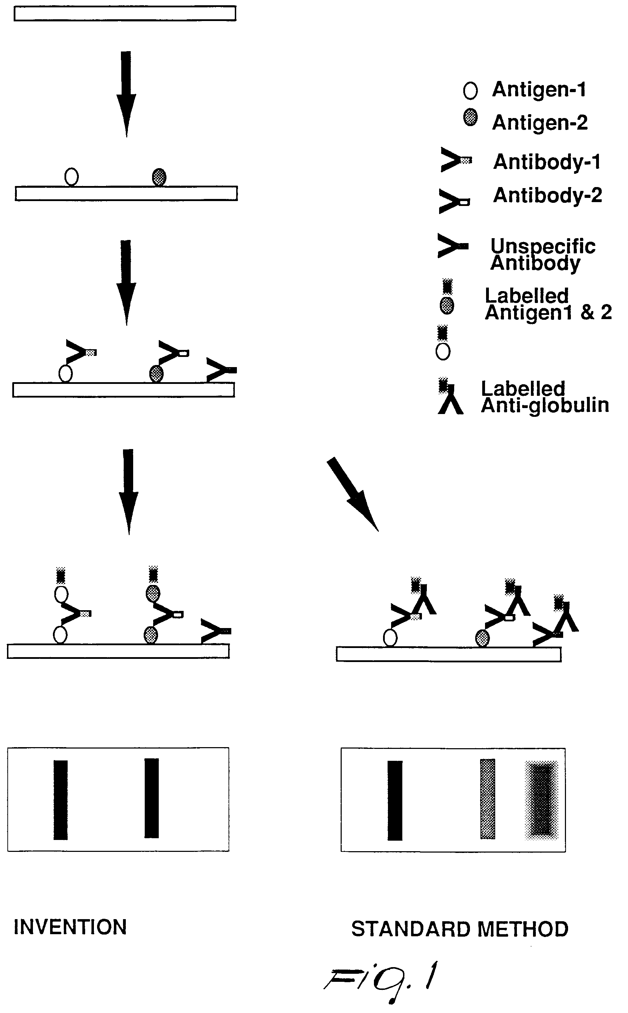

An assay system was designed to circumvent the problems of known screening and WB confirmatory tests. The present invention is a novel application of an immunoblot system suitable for the serodetection and confirmation of infection. Highly purified, diagnostically important antigens which are either purified from an infectious agent, recombinant DNA sources or synthetic peptides are immobilized on a solid support such that the antigens are spatially physically separated. A test sample suspected of containing antibody to one or more of these antigens is then contacted with the so-prepared matrix and allowed to incubate for a time and under conditions sufficient to form antigen/antibody complexes. The patient sample can be diluted prior to its contact with the matrix. Any antigen/antibody complexes bound to the matrix are detected by contacting one or more indicator reagents which comprise antibody-binding specific probes, such as antigens, conjugated to a signal generating compound which compound is capable of yielding a detectable measurable signal with the matrix, either sequentially or in a single reaction step, for a time and under conditions sufficient for antigen/antibody/indicator reagent complexes to form. The signal detected is representative of specifically bound antibody. This reaction procedure ("Invention") is shown in FIGURE 1, where it is compared to the presently-used method ("Standard Method").

The antigen-matrix can be prepared by the classical WB method, or by depositing antigens directly onto the solid-phase (solid support). Probes can be prepared either directly labelled with the signal generating compound (described hereinbelow) or labelled with a moiety which can be detected by a subsequent reaction with a second binding agent (i.e., biotin label with anti-biotin, biotin label with steptavidin). The use of a biotin/anti-biotin system for assays is the subject matter of published European Patent Application No. 0160900 (published November 13, 1985). Antigens can be naturally occurring as with a preparation of viral lysate; recombinant antigens or synthetic peptides also can be used. For convenience, the antigen designation "gp" means an antigen preparation which is glycosylated (glycoprotein), while the antigen designation "p" shall mean a non-glycosylated protein. It is known in the art that bacterial hosts such as E. coli produce non-glycosylated proteins, while hosts such as yeast can produce glycosylated proteins. Thus, the designation p41 means an antigen prepared recombinantly in a bacterial host such as E. coli, and the designation HIV-2 p36 means an antigen preparation prepared in a bacteria host such as E. coli. Also, the use of heterologous sourced antigens in the capture and probe phase is contemplated. For example, the immobilized (capture) antigen can be derived from a viral lysate and the probe made by recombinant DNA techniques or a synthetic oligopeptide.

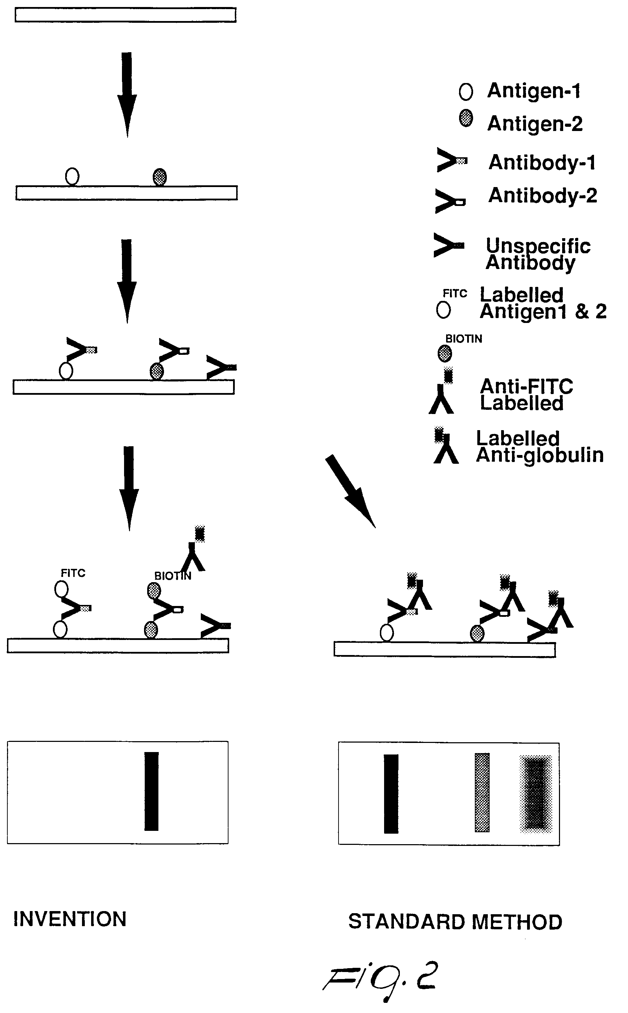

Further, the antigen-matrix preparation may be from more than one antigen source. For example, hepatotropic viruses such as antigens of HBV and HCV can be tested on the same solid phase. This methodology is shown in FIGURE 2. The specificity of the signal is determined by the probe, and not by the immobilized antigen. This embodiment of the present invention allows for the use of different labelling strategies in conjunction with the different probe antigens, thereby allowing"families" of antibodies to be detected.

A "specific binding member," as used herein, is a member of a specific binding pair. That is, two different molecules where one of the molecules through chemical or physical means specifically binds to the second molecule. Therefore, in addition to antigen and antibody specific binding pairs of common immunoassays, other specific binding pairs can include biotin and avidin, carbohydrates and lectins, complementary nucleotide sequences, effector and receptor molecules, cofactors and enzymes, enzyme inhibitors and enzymes, and the like. Furthermore, specific binding pairs can include members that are analogs of the original specific binding member, for example, an analyte-analog. Immunoreactive specific binding members include antigens, antigen fragments; antibodies and antibody fragments, both monoclonal and polyclonal; and complexes thereof, including those formed by recombinant DNA methods.

"Analyte," as used herein, is the substance to be detected which may be present in the test sample. The analyte can be any substance for which there exists a naturally occurring specific binding member (such as, an antibody), or for which a specific binding member can be prepared. Thus, an analyte is a substance that can bind to one or more specific binding members in an assay. "Analyte" also includes any antigenic substances, haptens, antibodies, and combinations thereof. As a member of a specific binding pair, the analyte can be detected by means of naturally occurring specific binding partners (pairs) such as the use of intrinsic factor protein in the capture and/or indicator reagents for the determination of vitamin B₁₂, or the use of a lectin in the capture and/or indicator reagents for the determination of a carbohydrate. The analyte can include a protein, a peptide, an amino acid, a hormone, a steroid, a vitamin, a drug including those administered for therapeutic purposes as well as those administered for illicit purposes, a bacterium, a virus, and metabolites of or antibodies to any of the above substances.

The test sample can be a mammalian biological fluid such as whole blood or whole blood components including red blood cells, white blood cells including lymphocyte or lymphocyte subset preparations, platelets, serum and plasma; ascites; saliva; stools; cerebrospinal fluid; urine; sputum; trachael aspirates and other constituents of the body which may contain or be suspected of containing the analyte(s) of interest. The test sample also can be a culture fluid supernatant, or a suspension of cultured cells. Mammals whose body fluids can be assayed for HIV antigen analyte or HIV antibody analyte according to the present invention include humans and primates, as well as other mammals who are suspected of containing these analytes of interest. It also is contemplated that non-biological fluid samples can be utilized.

The indicator reagent comprises a signal generating compound (label) conjugated to a specific binding member of each analyte. The indicator reagent produces a detectable measurable signal at a level relative to the amount of the analyte in the test sample. Each indicator reagent, while comprising a specific binding member of a different analyte, is conjugated to either the same or a different signal generating compound (label), which is capable of generating a detectable signal. In general, the indicator reagent is detected or measured after it is captured on the solid phase material. It is contemplated that different signal generating compounds can be utilized in the practice of the present invention. Thus, for example, different fluorescent compounds could be utilized as the signal generating compounds, one for each indicator reagent, and detection could be determined by reading at different wavelengths. Or, a short-lived chemiluminescent compound such as an acridinium or phenanthrindium compound and a long-lived chemiluminescent compound such as a dioxetane can be utilized to generate signals at different times for different analytes. Methods which detail the use of two or more chemiluminescent compounds which are capable of generating signals at different times are the subject matter of co-pending patent application U.S. Serial No. 636,038, which enjoys common ownership and is incorporated herein by reference. Acridinium and phenanthridinium compounds are described, for example, in European Patent Application No. 0 273 115, published July 6, 1988.

In addition to being either an antigen or an antibody member of a specific binding pair, the specific binding member of the indicator reagent can be a member of any specific binding pair, including either biotin or avidin, a carbohydrate or a lectin, a complementary nucleotide sequence, an effector or a receptor molecule, an enzyme cofactor or an enzyme, an enzyme inhibitor or an enzyme, and the like. An immunoreactive specific binding member can be an antibody, an antigen, or an antibody/antigen complex that is capable of binding either to the analyte as in a sandwich assay, to the capture reagent as in a competitive assay, or to the ancillary specific binding member as in an indirect assay. If an antibody is used, it can be a monoclonal antibody, a polyclonal antibody, an antibody fragment, a recombinant antibody, an anti-idiotype antibody, a chimeric antibody, a mixture thereof, or a mixture of an antibody and other specific binding members. The details of the preparation of such antibodies and their suitability for use as specific binding members are well known to those in the art.

The signal generating compound (label) of the indicator reagent is capable of generating a measurable signal detectable by external means. The various signal generating compounds (labels) contemplated include chromogens; catalysts such as enzymes, for example, horseradish peroxidase, alkaline phosphatase and B-galactosidase; luminescent compounds such as fluorescein and rhodamine; chemiluminescent compounds such as acridinium compounds, phenanthridinium compounds and dioxetane compounds; radioactive elements; colorimetric compounds such as metals (gold, silver, nickel and iron, for example) and direct visual labels. The selection of a particular label is not critical, but it will be capable of producing a signal either by itself or in conjunction with one or more additional substances. A variety of different indicator reagents can be formed by varying either the label or the specific binding member.

The capture reagents of the present invention comprise a specific binding member for each of the analytes of interest which are attached to the solid phase and which are unlabelled. In a most preferred embodiment, the antibody member of a specific binding pair is the analyte being detected, and the antigen member of a specific binding pair member is the capture reagent. The capture reagent can be directly or indirectly bound to a solid phase material before the performance of the assay, thereby enabling the separation of immobilized complexes from the test sample. This attachment can be achieved, for example, by coating the capture reagent specific binding member onto the solid phase by absorption or covalent coupling. Coating methods, and other known means of attachment, are known to those in the art.

The specific binding member of the capture reagent can be any molecule capable of specifically binding with another molecule. The specific binding member of the capture reagent can be an immunoreactive compound such as an antibody, antigen, or antibody/antigen complex. If an antibody is used, it can be a monoclonal antibody, a polyclonal antibody, an antibody fragment, a recombinant antibody, a mixture thereof, or a mixture of an antibody and other specific binding members.

While the number of antigens or epitopes of antigens that may be analyzed limited only by the size of the support, their variety and composition are limitless. For example, antigens derived from one or more of a viral lysate, recombinant protein and/or peptide can be combined in a single assay. Antigens having similar sizes but isolated from different sources are possible because they may be transferred from different gels to different discrete regions (sites) on the solid support. Reduction-sensitive or reduction-dependent antigens are conveniently handled by resolving them in separate preparative gels. In addition, the quantity of each antigen, the signal generating compound used and the probe antigen may be controlled and balanced by one of ordinary skill in the art to optimize assay selectivity and sensitivity.

Any desired number of antigens of one infectious agent, or combinations of antigens of several infectious agents, or any desired number of immunoglobulins of one infectious agent, or combinations of immunoglobulins of several infectious agents may be included on a single solid support and then analyzed in a single test procedure. This invention may be used to monitor concentrations of antibodies, or detect antigens which are normally endemic, but which may vary in a pathological condition, or to detect and quantitate antibodies or antigens which only are for a pathological condition. The present format also lends itself readily to use in combination assays which allow for the simultaneous detection of a panel of multiple antigens or antibodies, such as antigens or antibodies of HTLV-1, HBV, HCV, HEV, CMV, HSV, HPV, etc., bacterial antigens or antibodies, fungal and other microbiological antigens and antibodies. Such combinations are ones of preference which can be determined by the routineer.

It is contemplated and within the scope of the invention that the solid phase may comprise any suitable porous material with sufficient porosity to allow access by detection antibodies and a suitable surface affinity to bind antigens. Microporous structures are generally preferred, but materials with gel structure in the hydrated state may be used as well. Such useful solid supports include: Natural polymeric carbohydrates and their synthetically modified, cross-linked or substituted derivatives, such as agar, agarose, cross-linked alginic acid, substituted and cross-linked guar gums, cellulose esters, especially with nitric acid and carboxylic acids, mixed cellulose esters, and cellulose ethers; natural polymers containing nitrogen, such as proteins and derivatives, including cross-linked or modified gelatins; natural hydrocarbon polymers, such as latex and rubber; synthetic polymers which may be prepared with suitably porous structures, such as vinyl polymers, including polyethylene, polypropylene, polystyrene, polyvinylchioride, polyvinyl difluoride (PVDF), polyvinylacetate and its partially hydrolyzed derivatives, polyacrylamides, polymethacrylates, copolymers and terpolymers of the above polycondensates, such as polyesters, polyamides, and other polymers, such as polyurethanes or polyepoxides; porous inorganic materials such as sulfates or carbonates of alkaline earth metals and magnesium, including barium sulfate, calcium sulfate, calcium carbonate, silicates of alkali and alkaline earth metals, aluminum and magnesium; and aluminum or silicon oxides or hydrates, such as clays, alumina, talc, kaolin, zeolite, silica gel, or glass (these materials may be used as filters with the above polymeric materials); and mixtures or copolymers of the above classes, such as graft copolymers obtained by initializing polymerization of synthetic polymers on a pre-existing natural polymer. All of these materials may be used in suitable shapes, such as films, sheets, or plates, or they may be coated onto or bonded or laminated to appropriate inert carriers, such as paper, glass, plastic films, particles such as beads or fabrics.

The porous structure of nitrocellulose has excellent absorption and adsorption qualities for a wide variety of reagents including monoclonal antibodies. Nylon and PVDF also possesses similar characteristics and also are suitable.

It is contemplated that such porous solid supports described hereinabove are preferably in the form of sheets of thickness from about 0.01 to 0.5 mm, preferably about 0.1 mm. The pore size may vary within wide limits, and is preferably from about 0.025 to 15 microns, especially from about 0.15 to 15 microns. The surfaces of such supports may be activated by chemical processes which cause covalent linkage of the antigen or antibody to the support. The irreversible binding of the antigen or antibody is obtained, however, in general, by adsorption on the porous material by poorly understood hydrophobic forces.

A procedural control, for example, one that contains goat anti-human IgG included on the solid support to serve as an internal control to validate each positive reaction on the solid support indicates that a test sample was added a all reagents used in the subsequent incubation steps were added correctly and functioned properly. A positive procedural control is recommended for interpretation of the reactivities which occur on the solid support. It is also recommended that a reference spot coated with inert material, for example, casein acid hydrolysate is included on the solid support.

Positive and negative controls can be included in the assay of the present invention to ensure reliable results. A blank solid phase(s), to which no capture reagent has been attached, can be utilized as the negative reagent control. Positive controls can include a positive control for each analyte which control is tested separately, and a combined positive control wherein the presence of all analytes to be detected in the assay are determined. Also, such controls can be used to aid in quantitation of antibodies.

As previously stated, recombinantly-prepared antigens may be used in the assay instead of inactivated whole virus which has been cultured in a cell line capable of virus replication. Viral lysate reagents have an additional drawback of potentially dangerous manufacturing methods, since culturing of live virus in vitro and isolation and deactivation processes can expose workers to the infectious virus(es). The use of recombinantly-produced protein (antigen) as an antigen reagent in assay methods may solve these problems. Recombinant proteins are non-infectious and therefore the production and isolation of such proteins would be safer than culturing the whole virus. Also, the use of pure viral protein obtained by recombinant methods should eliminate some of the false positive reactions due to nonspecific reactions with contaminating proteins. Further, standardization of reagents should improve the specificity and predictive value of the assay. Recombinant proteins can be prepared in a variety of microbiological systems, including E. coli and yeast. It is recognized by those skilled in the art that glycosylated proteins are produced in yeast systems, and that non-glycosylated proteins are produced in E. coli.

Expression of HIV gp41 or parts of HIV gp41, for example, have demonstrated the utility of recombinant DNA (rDNA) derived HIV envelope sequences in diagnostic assays. Wood et al., Cold Spring Harbor Symposium on RNA Tumor Viruses, Cold Spring Harbor, New York, May 22-26 (1985); Chang et al., Biotechnology 3:905-909 (1985); Crowl et al., Cell 41:979-986 (1985); Cabradilla et al., Biotechnology 3:128-133 (1986). While it is general knowledge that viral proteins expressed in E. coli or other organisms have potential utility in diagnostic assays, development of immunoassays using these reagents, which also will have the specificity and sensitivity equal to or greater than the native viral proteins derived from the cell culture has been a difficult task. Further, the expression of HIV gag proteins in E. coli have indicated that the HIV gag proteins produced by rDNA technology could have potential diagnostic value. Wood et al., Cold Spring Harbor Symposium on RNA Tumor Viruses, Cold Spring Harbor, New York, May 22-26 (1985); Dowbenko et al., PNAS USA 82:7748-7752 (1985); Ghrayeb et al., DNA 5:93099 (1986); Steimer et al., Virology 150:283-290 (1986).

The present invention contemplates the use of recombinantly-produced HIV envelope proteins as assay reagents. The cloning of the HIV genome and expression of HIV envelope and core protein in E. coli, the purification and characterization of gp41 and p24, are known to those of ordinary skill in the art. Briefly, HIV-infected HT-9 cells are harvested and total cellular DNA is isolated and subjected to digestion. The DNA segments encoding for the core protein and for the envelope glycoprotein are further subcloned into bacterial expression vectors using well-known recombinant technology. Further, the detection of HIV-1 antibody by the use of recombinant antigens as the capture reagent and the indicator reagent allows for the detection of anti-HIV-1 antibodies of different immunoglobulin classes. These immunoglobulin classes include IgG, IgA, IgE and IgM. The detection of anti-HIV-1 IgG, IgM and IgA using the Abbott HIVAB® HIV-1/HIV-2 (rDNA) EIA assay has been described in an abstract by J. L. Gallarda et al., 5th Annual Forum on AIDS, Hepatitis and Other Blood-Borne Diseases, Atlanta, Georgia, March 29-April 1, 1992.

It is contemplated and within the scope of the present invention that recombinant antigens produced in heterologous sources can be utilized in the assay and will contribute an even greater lessening of false positive results. For example, if an E.coli prepared recombinant antigen such as HIV-1 p41 is used as the capture reagent, then a recombinant antigen (HIV-1 p41 or HIV-1 gp41) produced in any suitable source different to E. coli, such as in a suitable yeast host or other suitable host such as B. megaterium, can be used. The use of heterologous sources of antigens in assays , including recombinant antigens, is the subject matter of EP publication No. 0 313 986 published May 3, 1989.

Further, although the present invention preferably utilizes recombinantly produced antigens, it is well within the scope of the invention to utilize synthetic proteins instead of recombinantly produced antigens. Thus, various synthetically prepared peptides, of varying length, can be used. After the antigens are attached to the solid phase, the so-prepared solid phase can be "blocked" with a protein coating such as inactivated bovine serum albumin, inactivated horse serum or fish gelatin. Blocking masks potential non-specific binding sites on the binding layer. Suitable blocking conditions for each analyte/signal generating system/probe can vary and are determinable by those of ordinary skill in the art; usually, blocking is obtainable during an incubation period of about 1 hour at approximately 37°C.

The present invention also is adaptable to automated means, such as that provided by the Abbott Matrix™ Analyzer, available from Abbott Laboratories, Abbott Park, IL, USA. Generally in this automated method, antigens are immobilized individually in an array of dots separated by moats of air space on a sheet of nitrocellulose backed by inert plastic. These antigen-dotted nitrocellulose cards are housed in individual reaction cartridges and then incubated at 22° to 37°C with a test sample for a time and under conditions sufficient to detect specific antibodies which may be present in the test sample. Using a biotin-anti-biotin (BAB) amplifying immunodetection system optimized for maximal sensitivity as the conjugated signal-generating system capable of yielding a quantitatively measurable signal, specific antibodies present in the test sample to the antigens(s) immobilized on the solid support are simultaneously detected. These reaction cartridges containing the test cards are processed in an analyzer, thus rendering the incubations and washings semi-automated. The intensities of the color reaction are automatically quantitated by a reflectance reader which measures the density of reflectance (Dr), and the results are printed out in a digital format. Results are determined by comparing the Dr value(s) obtained from the test specimen to the Dr value(s) obtained from a normal negative population. Bar codes on the cartridges are used to assure positive test sample identification. The semi-automatic nature of the Abbott Matrix™ assay system can reduce hands-on time as well as assay time. The assay system using an antigen capture and antibody conjugate are the subject matter of U.S. Patent No. 5,120,662.

The present invention will now be described by way of examples, which are meant to illustrate, but not to limit, the spirit and scope of the invention.

### EXAMPLES

The following abbreviations are used throughout the Examples: BSA (bovine serum albumin); SDS(sodium dodecyl sulfate); APS (ammoniumperoxosulfate); PAGE (polyacrylamide gel electrophoresis); TEMED (N, N, N', N'-Tetramethylethylenediamine); MW (molecular weight); TBS (TRIS buffered saline); EDTA (Ethylenediaminetetraacetic acid); NC (negative control); PC (positive control); WB (Western blot); DMF (dimethyl formamide); AEC (3-amino-9-ethylcarbazole); and µl (microliter).

### Example 1. Preparation of HIV antigen matrix

The separation of viral lysates was performed by SDS-PAGE slab gel electrophoresis as described by Laemmli, Nature 227:680-685 (1970), using a 3% acrylamide stacking gel with a 10% resolving gel, both containing 0.1% SDS.

380 µl HIV-1 viral lysate diluted with the equal volume of nonreducing sample buffer (380 µl of 63 mM TRIS pH 6.8, 15% [v/v] glycerol, 3.38% SDS and 0.0025% bromphenolblue) and 55 µl of HIV-2 viral lysate diluted with an equal volume of reducing sample buffer (55 µl of 63 mM TRIS pH 6.8, 15% glycerol, 3.38% SDS, 5% 2-mercaptoethanol and 0.0025% bromphenolblue). The lysate sample buffer mixture was boiled for five minutes at 95°C, applied to the gel, and then electrophoresed for two and one-half hours with a constant current of 60 mA. Prestained low range MW markers with known molecular weights were run in the same gels and were used to estimate the molecular weight of the HIV-1 and HIV-2 lysate proteins.

The separated antigen matrix was obtained by transferring the antigens from the SDS-PAGE gels onto nitrocellulose/PVDF/Nylon matrices by using a modification of the WB technique as described by Towbin et al., Proc. Natl. Acad. Sci. USA 76:4350-4354 (1979). After transfer of the antigens to the matrices, excess protein binding sites were blocked by soaking in TRIS buffer (10 mM, pH 7.5) containing 154 mM NaCl and 5% (w/v) BSA.

### Example 2. Sample Treatment

Each test sample was diluted in TRIS buffer (20 mM, pH 8.3) containing 3% BSA, 0.2% Tween®-20, 0.1% NaN₃ and 1 mM EDTA prior to the overnight incubation at room temperature with the antigen matrix. Following overnight incubation, the matrix then was rinsed in PBS prior to reaction with the specific antibody binding probes or in the standard method with labelled goat anti-human IgG.

### Example 3. Preparation of Specific Antibody Binding Probes

Recombinant antigens representing the HIV-1 proteins p24 and gp41, and HIV-2 protein gp36 were expressed in E. coli Blue XL-1 using standard techniques known to those of ordinary skill in the art. E. coli Blue XL-1 is commercially available from, for example, Stratagene Corp., La Jolla, CA, USA, under the product name of Stratagene Cloning System. These proteins (now designated as HIV-1 p24, HIV-1 p41 and HIV-2 p36) then were individually conjugated to horseradish peroxidase (HRPO) using the technique described by Nakane et al., J. Histochem. Cytochem. 22:1084-1091 (1974). Following this conjugation reaction, the resultant enzyme-protein mixture was purified by column chromatography using a Sephacryl®S-300 or S-200 column.

### Example 4. HRPO Detection

### (a) Colorimetric.

HRPO substrates were prepared as follows: 4-chloro-1-naphthol dissolved in methanol was diluted with acetate buffer (50 mM, pH 5.0), or alternatively AEC dissolved in DMF (dimethylformamide) then diluted with acetate buffer. Peroxide was added to these substrate solutions immediately before the detection procedure. The reaction was stopped by aspirating the solution. The membrane was then rinsed with demineralized water. The colored matrix was photographed using instant film (available from Polaroid Corp., Cambridge, MA, USA).

### (b) Chemiluminescent.

A luminol-peroxide enchancer mixture (available from Amersham, Braunschweig, Germany, as an ECL protein detection kit) was used according to the manufacturer's instructions. The chemiluminescent signal was detected using X-ray film (available from Kodak Corp, Rochester, NY, USA). After immersing and incubating the matrix in luminol, the excess substrate was drained from the matrix, and then placed in direct contact with X-ray film for exposures.

### Example 5. Detection

### (a) Standard Method.

The antigen/antibody matrix processed as described in Examples 1 and 2, was incubated with biotin labelled-goat anti-human IgG (Pierce Immunopure™, available from Pierce Inc., Rockford, IL, USA) diluted 1:1250 in TBS containing 10% NCS (newborn calf serum) for two hours at room temperature. Following washing of the matrix in TBS and Tween®-20 buffer, the matrices were incubated for 15 minutes with peroxidase labelled Streptavidin (available from Pierce, Inc., Rockford, IL, USA) diluted 1:500 in TBS. Following washing with TBS, the bound HRPO was detected using the substrate solution consisting of AEC and H₂O₂.

### (b) Present Invention Method

The antigen/antibody matrix processed as described in Examples 1 and 2 was incubated for 2 hours at room temperature with HRPO-labelled antigens diluted in a conjugate diluent (TBS containing 10% NCS) at a concentration of 10 µg/ml. Following washing with TBS, the bound HRPO was detected as specified in Example 5(a).

### Example 6. HIV-1 Lysate

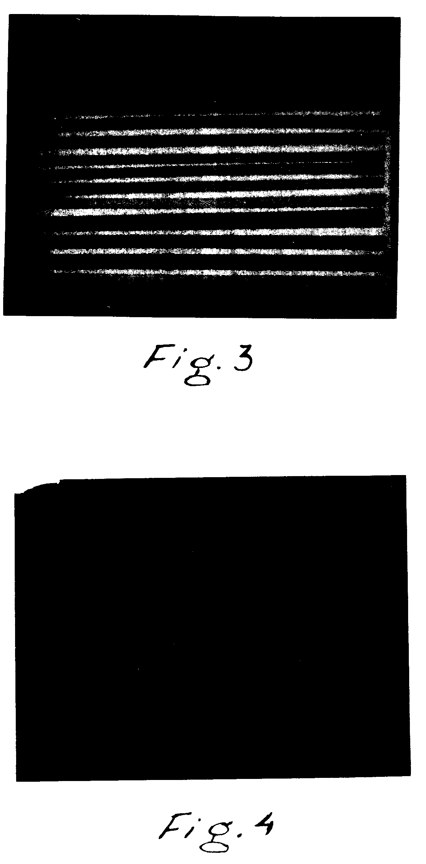

Using HIV-1 lysate prepared as described in Example 1 to make the separated antigen matrix and the samples prepared as described in Example 2, 11 experiments were performed according to the procedures outlined in Examples 4 and 5 simultaneously and as follows. The results of these experiments are shown in FIGURE 3. Matrix numbers 1, 3, 5 and 9 were reacted with HIV-1 negative samples, and matrices 2, 4, 6, 8, 10 and 11 were reacted with a sample obtained from an HIV-1 positive patient (WHO standard). Detection of bound antibody on matrices 1 to 4 was by the standard method of detection as described in Example 5(a) using goat anti-human IgG as described hereinabove. Specific detection of bound antibody was performed as described in Example 5(b) by using the antigen probes for HIV-1 gp41 prepared as described in Example 3 in matrices 5 to 8, and using the HIV-1 p24 antigen prepared as described in Example 3 in matrices 9 to 11. Referring to FIGURE 3, the results demonstrate the unexpected nature of the invention with almost no detectable background in the specific probe method (matrices 5 to 9) compared to the standard method (matrices 1 and 3). The high specificity of the method of the invention is demonstrated as only the antibodies which specifically react with the probes are detected in matrices 6 to 8 and 10 and 11, unlike the standard method which shows all populations with no differentiation.

### Example 7. HIV-2 Lysate

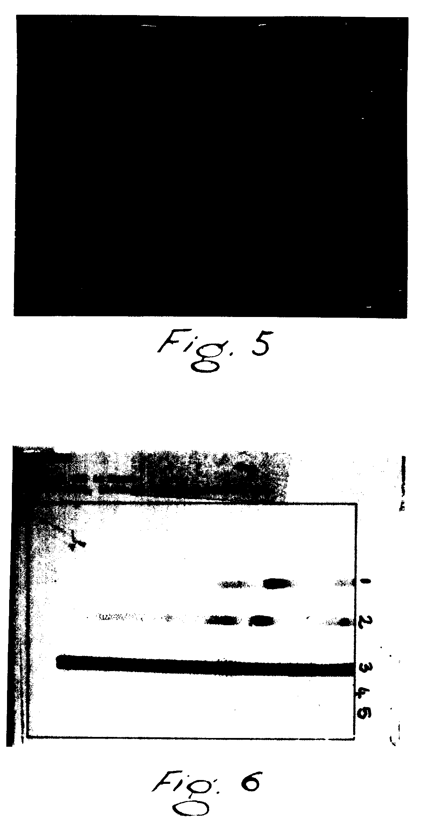

Example 6 was repeated except that HIV-2 lysate prepared as described in Example 1 was used to make the antigen matrices, and a positive sample was obtained from a patient with an HIV-2 infection. Matrix numbers 1, 5 and 9 were reacted with HIV-2 negative samples, while matrices 2 to 4, 6 to 8 and 10 to 12 were reacted with the HIV-2 positive sample. Matrix 1 was the negative control for the standard test; matrix 6 was the negative control for the method of the invention. Matrices 7 and 8 were assayed using a biotin enhancement system as described hereinabove. Detection of bound antibody on matrices 1 to 4 was performed by using the standard method described in Example 5(a) using goat anti-human IgG described hereinabove. The results of this example are shown in FIGURE 4. Again, the results demonstrate the superior specificity of the invention over the standard method. Matrix 1 (negative control) tested by the standard method shows visible high background signals as well as a non-specific band. The matrices tested by the method of the invention (matrices 5 to 12) only show specificity for anti-HIV-2 antibodies with effectively no background.

### Example 8.Detection with HRPO or a Chemiluminescent Label

HIV-1 and HIV-2 lysate prepared as described in Example 1 and test sample prepared as described in Example 2 was used to prepared three matrices. Matrices 1 and 2 then were reacted with an HIV-1 positive sample as described in Example 6. Matrix 3 was reacted with an HIV-2 positive sample as described in Example 7. Matrix 1 and 2 were subject to specific probe detection using the p41-HRPO probe prepared as described in Examples 3 and 4, and detected as described in Example 5(b), while Matrix 3 was tested using the standard method as described in Example 5(a). The colorimetric results are shown in FIGURE 5. As seen in FIGURE 5, the results again demonstrate that the method of the invention has high specificity.

Next, the same three matrices were used, but this time these matrices were tested using the chemiluminescence method described in Example 4(b). The results are shown in FIGURE 6. Referring to FIGURE 6, matrices 1 and 2 were subjected to the method of the invention as described in Example 4(b) and 5(b), and matrix 3 was subjected to the standard method as described in Example 4(b) and 5(a). Matrices 4 and 5 were negative system controls. The results demonstrate that a strong specific signal was produced by using the inventive method, while the standard method had backgrounds so high that it was not possible to distinguish the specifically bound signals.

## Claims

1. A method for detecting one or more specific antibodies in a test sample, comprising:
(a) contacting a test sample with a solid phase matrix containing more than one antigen, wherein said antigens are spatially physically separated from each other on said matrix, for a time and under conditions sufficient to form antigen/antibody complexes;
(b) contacting said antigen/antibody complexes of step (a) with an indicator reagent wherein said indicator reagent comprises a probe specific for said antibody being detected, conjugated to a signal generating compound capable of generating a detectable measurable signal;
(c) measuring the generated signal to determine the presence of the antibody in the test sample.

2. The method of claim 1, wherein the antigens are selected from the group consisting of a lysate, a recombinant protein and a synthetic peptide.

3. The method of claim 2 wherein said lysate is an HIV lysate containing antigens selected from the group consisting of HIV-1 p24, HIV-1 gp41, HIV-1 p41, HIV-2 gp36 and HIV-2 p36.

4. The method of claim 1, wherein said probe is selected from the group consisting of a lysate, a recombinant protein and a synthetic peptide or a combination thereof.

5. The method of claim 1, wherein each said indicator reagent capable of detecting each said antigen is labelled with a different signal generating compound.

6. The method of claim 5, wherein said signal generating compound is selected from the group consisting of a luminescent compound, a chemiluminescent compound, an enzyme, a colorimetric and a chromogenic compound

7. The method of claim 6 wherein said signal generating compound further comprises a hapten-anti-hapten binding pair selected from the group consisting of biotin/anti-biotin, biotin/steptavidin and fluorescein/anti-fluorescein.

8. A test kit for detecting one or more more antibodies in a test sample comprising
(a) a capture antigen specific for the antibody to be detected;
(b) an indicator reagent comprising a probe specific for the antibody to be detected conjugated to a signal generating compound capable of generating a detectable measurable signal.

9. The test kit of claim 8 wherein said antigen is selected from the group consisting of a lysate, a recombinant protein and a synthetic peptide, and wherein said lysate is an HIV lysate containing antigens selected from the group consisting of HIV-1 p24, HIV-1 gp41, HIV-1 p41, HIV-2 gp36 and HIV-2 p36.

10. The test kit of claim 8 wherein said signal generating compound is selected from the group consisting of a luminescent compound, a chemiluminescent compound, an enzyme, a colorimetric and a chromogenic compound.
